# EUROPEAN PATENT APPLICATION

(11) **EP 4 696 276 A1**
(43) Date of publication of application: **18.02.2026**
(21) Application number: 25195208.1
(22) Date of filing: 11.08.2025
(51) Int. Cl.: A61B 34/30, A61B 17/92

(54) **END EFFECTOR WITH INSTRUMENT INTERFACE FOR HIP PROCEDURE**

(30) Priority: 13.08.2024 US 202418802689
(71) Applicant: Globus Medical, Inc., Audubon, PA 19403 (US)
(72) Inventor: Stoudmann, Joel, 1012 Lausanne (CH); Oluwasakin, Israel, Dresher, 19025 (US); Kozakiewicz, Kornel, 1024 Ecublens (CH); Eckert, Peter, 1020 Renens (CH); Cameron, Hayden, Philadelphia, 19129 (US); Chappuis, Olivier, 1095 Lutry (CH)
(74) Representative: Morabito, Sara

(57) **Abstract**

Various implementations include an end effector for a total hip arthroplasty (THA) procedure. An example end effector can include: an adapter for holding a surgical instrument, the adapter having an elongate body and a projection extending therefrom; a main body having: a proximal end including an interface for attaching to a robot arm; and a distal end including an elongate recess and a slot for connecting with the surgical instrument; and a connector adapted to secure the projection in the slot to securely attach the adapter to the main body.

## Description

### BACKGROUND OF THE INVENTION

The invention relates generally to devices, systems, and methods for use in surgical procedures. More particularly, the invention relates to devices, systems, and methods for performing total hip arthroplasty (THA) surgical procedures.

Hip arthroplasty, or hip replacement, is a surgical procedure used to resurface and reconstruct a hip joint that has been damaged by disease or injury, e.g., by arthritis or a fracture. THA devices replace both the acetabulum and the femoral head that collectively comprise the hip joint. An acetabular implant is secured to the acetabulum, forming a replacement articulating surface which interfaces with the femoral implant secured to the end of the femur. The femoral implant is pivotably coupled to the acetabular implant, thereby reconstructing the hip joint. Exemplary acetabular implants are disclosed in, e.g., US Patent Application No. 17/024,876, filed September 18, 2020 (published as US 2022/0087823 A1), which is incorporated by reference as though fully set forth herein.

Robotic surgery systems including computer-assisted navigation have become a well-established technique in operating rooms, including their use in arthroplasty procedures. Computer-assisted navigation systems provide surgeons with computerized visualization of how a surgical instrument or other device that is posed relative to a patient correlates to a pose relative to medical images of the patient's anatomy, and how those poses correlate to a pre-operative surgical plan. Camera tracking systems for computer assisted surgery navigation typically use a set of tracking cameras to track a pose of a reference element on the surgical instrument, which may be coupled to a surgical robot and may be positioned by a surgeon during surgery, relative to a patient reference element (or "dynamic reference base" (DRB)) affixed to the patient. A computer model of a real instrument is associated with a reference element, so that the computer model can be overlaid on registered images of patient's anatomy. The camera tracking system uses the relative poses of the reference elements to determine how the real instrument is posed relative to the patient and to determine how the computer model of the real instrument is to be correspondingly posed as overlaid on the medical images. The surgeon can thereby use real-time visual feedback of the relative poses to navigate the surgical instrument during a surgical procedure on the patient.

As noted above, a robotic system may be used for arthroplasty procedures. The robotic system (or, "robot" or "surgical robot") has a serial arm on which an end effector is mounted. The surgeon (or "user") may hold the end effector or any instruments coupled thereto, to perform surgical operations while watching in real time on a navigation system (e.g., on stand-alone display(s) or an Augmented Reality (AR) headset), and to receive various types of relevant feedback and information associated with a defined plan for and/or progress of the surgical procedure.

The serial arm can move through computer guided control to a suitable position for the surgery, e.g., pursuant to the surgeon's request, which may be provided via a foot pedal, touchscreen, AR interaction, etc. The passive robotic structure allows the surgeon to precisely perform each operation in the procedure.

Various workflows can be available for use with the system. Such workflows may incorporate preoperative scans or images of the patient (e.g., x-ray or Computerized Tomography (CT)). On the other hand, other workflows may be imageless, and may not require any pre-operative images. Some workflows may incorporate acquisition of intra-operative information about the patient anatomy. In one example, the surgeon may measure key parameters of the bone using a camera tracking system and an appropriate tracked instrument to capture points on patient anatomy. Later, this information, and other intra-operatively-acquired information may be used to plan the implant position and orientation with respect to patient anatomy, and to navigate the robot and surgical instruments during the surgical procedure.

In some workflows, the surgeon may rigidly attach a reference element to one or more bones, where the reference element includes fiducials which are detected by tracking cameras for computer assisted navigation. The reference elements allow tracking of bone position by the navigation system. The reference elements can be positioned on the bone and oriented such that they can be seen by the tracking cameras of the navigation system. Once positioned, the reference elements are attached with fixation structures (e.g., screw pins, "crocodile" jaws) on the bone (e.g., pelvis or femur). The reference elements' respective positions and orientations stay rigidly fixed with respect to the bone throughout the procedure.

Another process of various workflows is to register the patient in the tracking space of the navigation system. Patient registration can include matching the patient anatomy with a numeric representation of the corresponding bone, such as a three-dimensional (3D) model of the bone. The bone representation may be constructed from, e.g., a set of CT images (CT workflow), a set of fluoroscopy images, or based on a generic bone model (imageless workflow).

Although current surgical approaches offer sophisticated techniques in robotic and navigation-assisted surgeries, current approaches for bone preparation, trialing, and implant placement may have shortcomings, e.g., in THA procedures.

### BRIEF DESCRIPTION OF THE INVENTION

A first aspect of the disclosure provides an end effector for a total hip arthroplasty (THA) procedure, the end effector including: an adapter for holding a surgical instrument, the adapter having an elongate body and a projection extending therefrom; a main body having: a proximal end including an interface for attaching to a robot arm; and a distal end including an elongate recess and a slot for connecting with the surgical instrument; and a connector adapted to secure the projection in the slot to securely attach the adapter to the main body.

According to certain embodiments, the connector and the elongate recess limit at least two degrees of freedom (DoF) of the surgical instrument.

According to certain embodiments, the surgical instrument and the adapter are configured to connect with the main body as a single unit. In certain implementations, the surgical instrument and adapter can be pre-coupled and connected with the main body using a quick-connect approach, e.g., with a single operator hand. In some of these cases, a final connection is made with a second operator's hand.

According to certain embodiments, the projection complements the slot in the main body.

According to certain embodiments, the projection includes a notch that complements the connector and enables attachment and detachment of the adapter from the main body. In some examples, the notch includes a V-shaped slot.

According to certain embodiments, when secured, the adapter interfaces with the elongate recess and is coupled with the connector.

According to certain embodiments, the adapter includes at least two limiters to limit degrees of freedom (DoF) of the surgical instrument. For example, translation and rotation can limited. In some examples, limiters can include buttons, protrusions, or other interfaces.

According to certain embodiments, the surgical instrument is of a first type of a plurality of types of surgical instruments configured to detachably couple with the main body. For example, types of surgical instruments can include a reamer, an impactor, etc.

According to certain embodiments, the surgical instrument includes an impactor.

According to certain embodiments, the connector enables decoupling of the impactor from the main body in a single disconnect process, restoring at least two DoF limited by the end effector. For example, in some cases, the operator (e.g., surgeon) may beneficially perform the single disconnect process to test the grip and/or fit of the implant in the pocket. In additional cases, the operator may beneficially perform the single disconnect process if the surgical robot fails or locks for any reason.

According to certain embodiments, decoupling the impactor from the main body enables manual manipulation of the impactor by a surgeon.

According to certain embodiments, the surgical instrument is configured to be coupled or decoupled from the main body at any stage of the THA procedure.

According to certain embodiments, the surgical instrument includes a navigation array.

According to certain embodiments, the elongate recess has one of a V-shaped cross-section or a U-shaped cross-section.

According to certain embodiments, the adapter is cylindrical and complements the elongate recess.

According to certain embodiments, the connector includes a pin sized for insertion or removal from a slot in the projection in the adapter. In a version the pin is dimensioned to be housed in the slot. In a version the pin is configured to be completely housed in the slot.

According to certain embodiments, when secured, the degrees of freedom (DoF) of the surgical instrument relative to the end effector is approximately zero.

According to certain embodiments, the interface on the proximal end includes a set of pins for interfacing with the robot arm. In one example, the set of pins includes two pins, three pins, four pins, or more. In a particular example, the set of pins includes three pins.

In a version the set of pins comprises at least one pin extending along a first axis and at least a second pin extending along a second axis perpendicular to the first axis. In a further version the set of pins comprises at firs plurality of pins extending along a first axis and a second plurality of pins extending along a second axis perpendicular to the first axis.

According to certain embodiments, the end effector further includes kinematic mounts proximate each of the pins.

According to certain embodiments, the interface includes a set of two U-shaped elements adjustably coupled by at least one screw.

According to certain embodiments, the connector includes an actuator enabling a user to couple or decouple the surgical instrument from the main body. In some cases, the actuator includes a handle.

According to certain embodiments, the end effector includes a drape across the interface on the proximal end of the main body, wherein the drape provides a sterile shield between the actuator and the surgical instrument.

According to certain embodiments, the sterile shield allows the user to couple or decouple the surgical instrument from the main body without compromising pre-established sterility.

According to certain embodiments, a method includes coupling the end effector to the surgical instrument.

According to certain embodiments, the method further includes decoupling the end effector from the surgical instrument without repositioning the robot arm. For example, the decoupling of the end effector can be performed without moving the arm out of the surgical field.

According to certain embodiments, a surgical robot includes the robot arm coupled with the end effector.

These and other aspects, advantages and salient features of the invention will become apparent from the following detailed description, which, when taken in conjunction with the annexed drawings, where like parts are designated by like reference characters throughout the drawings, disclose embodiments of the invention.

### BRIEF DESCRIPTION OF THE DRAWINGS

Aspects of the present disclosure are illustrated by way of example and are not limited by the accompanying drawings. In the drawings:
**FIG.** 1 is an overhead view of a surgical system arranged during a surgical procedure in a surgical room which includes a camera tracking system for computer assisted navigation during surgery, and a surgical robot for robotic assistance according to some embodiments of the present disclosure.
**FIG. 2** illustrates the camera tracking system and the surgical robot of **FIG. 1****,** positioned relative to a patient according to some embodiments of the present disclosure.
**FIG. 3** further illustrates the camera tracking system and the surgical robot of **FIGS. 1-2****,** configured according to some embodiments of the present disclosure.
**FIG. 4** illustrates a block diagram of a surgical system that includes an extended reality headset, a computer platform, imaging devices, and a surgical robot, which are configured to operate according to some embodiments of the present disclosure.
**FIG. 5** illustrates a flowchart of a workflow during an intra-operative portion of a total hip arthroplasty (THA) surgery, in accordance with some embodiments of the present disclosure.
**FIG. 6** illustrates a robot arm with an end effector for holding a surgical instrument, in accordance with some embodiments of the present disclosure.
**FIG. 7** illustrates a separated assembly including an end effector, adapter, surgical instrument, and navigation array, in accordance with some embodiments of the present disclosure.
**FIG. 8** illustrates the connected assembly of **FIG. 7****.**
**FIG. 9** illustrates a separated assembly including an end effector, adapter, surgical instrument, and navigation array, in accordance with some embodiments of the present disclosure.
**FIG. 10** illustrates a separated assembly including an end effector, adapter, and surgical instrument, in accordance with some embodiments of the present disclosure.
**FIG. 11** illustrates a separated assembly including an end effector and adapter, in accordance with some embodiments of the present disclosure.
**FIG. 12** illustrates an end view of a portion of an end effector, in accordance with some embodiments of the present disclosure.
**FIG. 13** illustrates a partial cross-sectional view of a portion of an end effector, in accordance with some embodiments of the present disclosure.
**FIG. 14** illustrates an end view of an interface on an end effector in accordance with some embodiments of the present disclosure.
**FIG. 15** and **FIG. 16** show perspective views of portions of an end effector and a surgical drape in accordance with some embodiments of the present disclosure.
**FIG. 17** and **FIG. 18** show distinct perspective views of a portion of an end effector coupled with a surgical instrument adapter, in accordance with some embodiments of the present disclosure.
**FIG. 19** illustrates a schematic view of a system, in accordance with some embodiments of the present disclosure.
**FIG. 20** and **FIG. 21** show distinct views of example end effector interfaces, in accordance with some embodiments of the present disclosure.

It is noted that the drawings of the disclosure are not necessarily to scale. The drawings are intended to depict only typical aspects of the disclosure, and therefore should not be considered as limiting the scope of the disclosure. In the drawings, like numbering represents like elements between the drawings.

### DETAILED DESCRIPTION OF THE INVENTION

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "attached," "supported," and "coupled" and variations thereof are used broadly and encompass both direct and indirect mountings, connections, attachments, supports, and couplings. Further, "connected" and "coupled" are not restricted to physical or mechanical connections or couplings.

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

The present application is related to (1) Patent App. No. 15/180,126, filed June 13, 2016 (U.S. 10,842,453), (2) Patent App. No. 15/157,444, filed May 18, 2016 (U.S. Pub. No. 2016/0256225), (3) Patent Application No. 18/743,685, filed June 14, 2024 (Docket ROBOT.143.0005), (4) Patent Application No. 18/743,388, filed June 14, 2024 (Docket ROBOT.143.0002), (5) Patent Application No. 18/743,647, filed June 14, 2024 (ROBOT.143.0004), (6) Patent Application No. 18/743,615, filed June 14, 2024 (Docket ROBOT.143.0003), (7) Patent Application No. 18/770,993, filed June 14, 2024 (Docket ROBOT.146.0002), and (8) Patent Application No. 18/770,993 (ROBOT.146.0002), each of which is incorporated herein by reference.

Robotic surgery systems and workflows associated therewith may provide improved outcomes in surgeries such as, e.g., THA surgeries compared to more traditional approaches. For example, robotic surgery systems may provide additional accuracy and force assistance when preparing the acetabulum, and additional accuracy and alignment when trialing and placing implants. In certain embodiments, e.g., including intra-operative CT imaging, confirmatory feedback regarding screw location may also be provided prior to drilling. Aspects of the disclosed embodiments are discussed below.

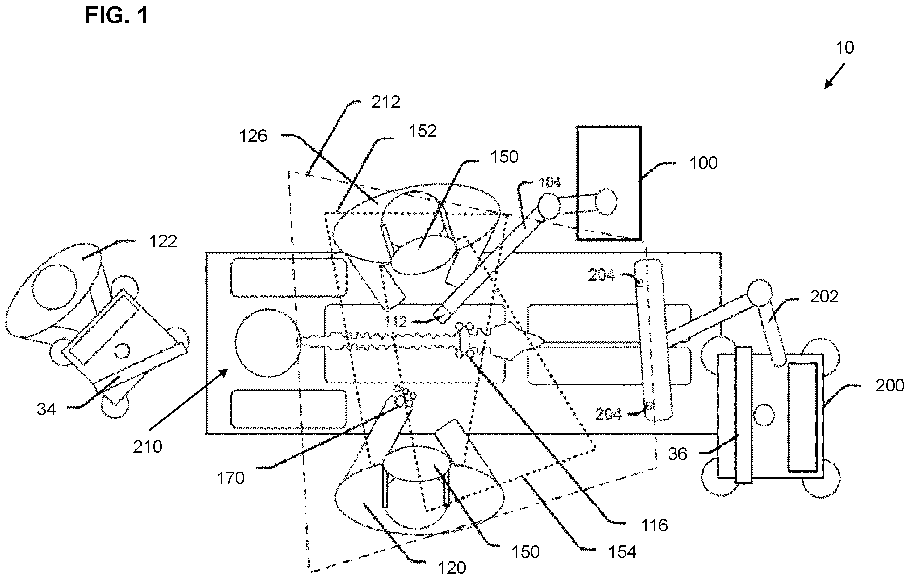

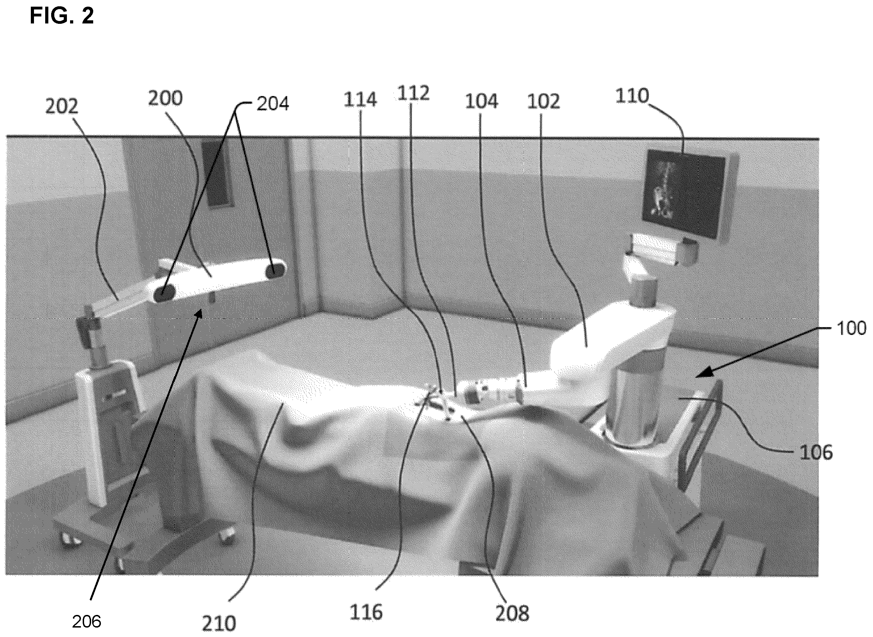

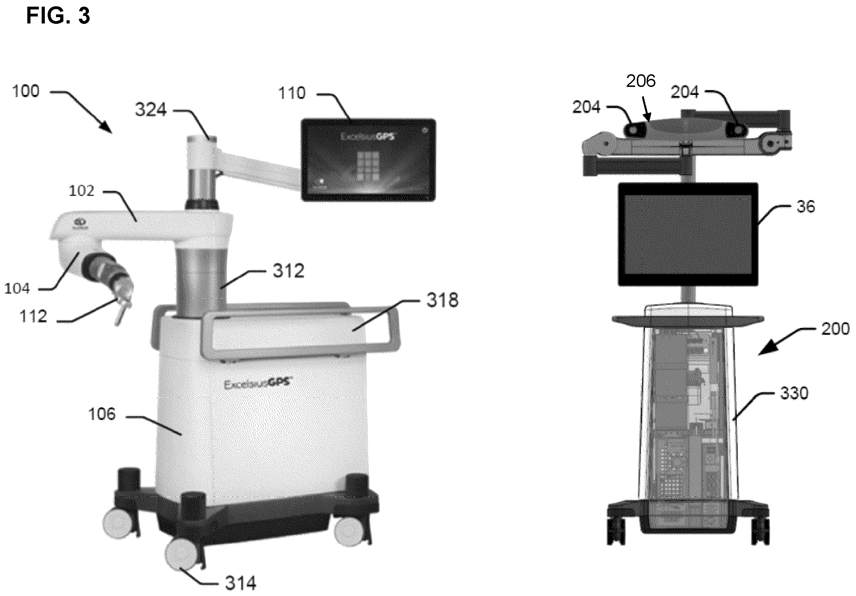

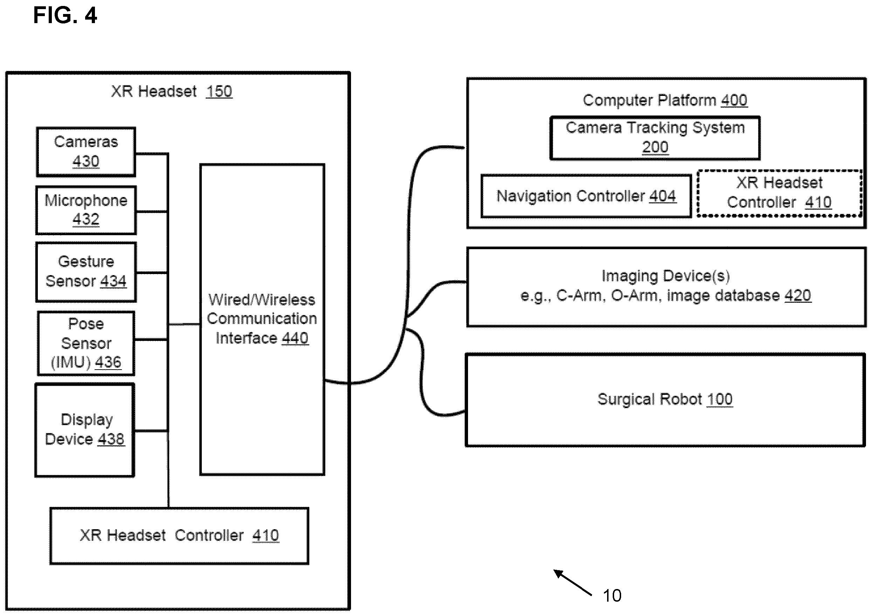

**FIG. 1** is an overhead view of a surgical system 10 arranged during a surgical procedure in a surgical or operating room. The system 10 includes a camera tracking system 200 for computer assisted navigation during surgery and may further include a surgical robot 100 for robotic assistance according to some embodiments. **FIG. 2** illustrates the camera tracking system 200 and the surgical robot 100 positioned relative to a patient according to some embodiments. **FIG. 3** further illustrates the camera tracking system 200 and the surgical robot 100 configured according to some embodiments. **FIG. 4** illustrates a block diagram of a surgical system 10 that includes an extended reality (XR) headset 150, a computer platform 400, imaging devices 420, and the surgical robot 100 which are configured to operate according to some embodiments.

The camera tracking system 200 **(****FIGS. 1-4****)** in some cases includes an intraoperative imaging system, that can include distinct imaging modalities. These imaging modalities may include one or more of fluoroscopy, 2D Radiography, and Cone-beam computed tomography (CBCT). Fluoroscopy is a medical imaging technique that shows a continuous X-ray image on a monitor, much like an X-ray movie. 2D Radiography is an imaging technique that uses X-rays to view the internal structure of a non-uniformly composed and opaque object such as the human body. CBCT (or, cone beam 3D imaging or C-arm CT), is a medical imaging technique consisting of X-ray computed tomography where the X-rays are divergent, forming a cone. The camera tracking system 200 is capable of: (1) capturing 3-Dimensional (3D) images (e.g., CT, CBCT, MCT, PET, Angiogram, MRI, ultrasound, etc.), (2) capturing 2-Dimensional (2D) images (e.g., fluoroscopy, digital radiography, ultrasound, etc.), and (3) containing an integrated or detachable navigation array having tracking markers (e.g., NIR retroreflective, NIR LED, visible, etc.), which is calibrated to the image space of the 2D and 3D images.

The surgical robot 100 is capable of: (1) using registered 2D and/or 3D images for surgical planning, navigation, and guidance in a variety of workflows (e.g., intraoperative 3D, intraoperative 2D, preoperative 3D to 2D, and intraoperative 3D to 2D, etc.); and (2) containing a camera tracking system 200 capable of tracking markers (e.g., NIR retroreflective, NIR LED, visible, etc.). In some cases, as noted herein, a dynamic reference base (DRB) (or patient reference array) 116 is (1) capable of rigidly attaching to the patient anatomy, and (2) contains an array of tracking markers (e.g., NIR retroreflective, NIR LED, visible, etc.).

The XR headsets 150 may be configured to augment a real-world scene with computer generated XR images while worn by personnel in the operating room. The XR headsets 150 may be configured to provide an augmented reality (AR) viewing environment by displaying the computer generated XR images on a see-through display screen that allows light from the real-world scene to pass therethrough for combined viewing by the user. Alternatively, the XR headsets 150 may be configured to provide a virtual reality (VR) viewing environment by preventing or substantially preventing light from the real-world scene from being directly viewed by the user while the user is viewing the computer-generated AR images on a display screen. The XR headsets 150 can be configured to provide both AR and VR viewing environments. Thus, the term XR headset encompasses both or either of an AR headset or a VR headset.

With continuing reference to **FIGS. 1-4****,** the surgical robot 100 may include, for example, one or more robot arms 102, 104, a display 110, an end effector 112, for example, including a guide tube 118, and an end effector reference element 114 which can include one or more tracking fiducials. A patient reference element (or DRB) 116 (shown in **FIG. 1****)** has a plurality of tracking fiducials and is secured directly to the patient 210. For example, a navigated pelvis DRB marker array may be placed intra-incision or extra-incision with the help of cortical pins drilled into the pelvic bone. In some embodiments, the DRB is oriented to be visible by the tracking camera(s) 204 (e.g., a stereoscopic tracking camera) installed on the camera tracking system 200 and/or the XR headset 150. A reference element 170 is attached to or formed on an instrument, surgical tool, surgical implant device, etc.

The camera tracking system 200 includes tracking cameras 204 which may be spaced apart to provide stereo cameras configured with partially overlapping fields-of-view. The camera tracking system 200 can have any suitable configuration of arm(s) 202 to move, orient, and support the tracking cameras 204 in a desired location, and may contain at least one processor operable to track the location of an individual fiducial and pose of an array of fiducials of a reference element.

As used herein, the term "pose" refers to the location (e.g., along three orthogonal axes, e.g., the x-, y-, and z-axes) and/or the rotation angle (e.g., about the three orthogonal axes) of fiducials (e.g., DRB) relative to another fiducial (e.g., surveillance fiducial) and/or to a defined coordinate system (e.g., camera coordinate system, navigation coordinate system, etc.). A pose may therefore be defined based on only the multidimensional location of the fiducials relative to another fiducial and/or relative to the defined coordinate system, based on only the multidimensional rotational angles of the fiducials relative to the other fiducial and/or to the defined coordinate system, or based on a combination of the multidimensional location and the multidimensional rotational angles. The term "pose" therefore is used to refer to location, rotational angle, or combination thereof of, e.g., an instrument reference element 170, a patient reference element 116, or the like.

The tracking cameras 204 may include, e.g., infrared cameras (e.g., bifocal or stereophotogrammetric cameras) operable to identify, for example, active and passive tracking fiducials for single fiducials (e.g., a surveillance fiducial) and reference elements which can be formed on or attached to the patient 210 (e.g., patient reference element or DRB 116), end effector 112 (e.g., end effector reference element 114), XR headset(s) 150 worn by a surgeon 120 and/or a surgical assistant 126, etc. in a given measurement volume of a camera coordinate system while viewable from the perspective of the tracking cameras 204. The tracking cameras 204 may scan the given measurement volume and detect light that is emitted or reflected from the fiducials in order to identify and determine locations of individual fiducials and poses of the reference elements in three-dimensions. For example, active reference elements may include infrared-emitting fiducials that are activated by an electrical signal (e.g., infrared light emitting diodes (LEDs)), and passive reference elements may include retro-reflective fiducials that reflect infrared light (e.g., they reflect incoming IR radiation into the direction of the incoming light), for example, emitted by illuminators on the tracking cameras 204 or other suitable device.

The XR headsets 150 may each include tracking cameras (e.g., spaced apart stereo cameras) that can track the location of a surveillance fiducial and poses of reference elements within the XR camera headset fields of view (FOVs) 152 and 154, respectively. Accordingly, as illustrated in **FIG. 1****,** the location of the surveillance fiducial and the poses of reference elements on various objects such as, e.g., instrument reference element 170 and patient reference element 116, can be tracked while in the FOVs 152 and 154 of the XR headsets 150 and/or a FOV 212 of the tracking cameras 204.

**FIGS. 1** **and** **2** illustrate a potential configuration for the placement of the camera tracking system 200 and the surgical robot 100 in an operating room environment. Computer assisted navigated robotic surgery can be provided by the surgical robot 100, the camera tracking system 200 controlling the XR headsets 150 and/or other displays 34, 36, and 110 to display surgical procedure navigation information.

The camera tracking system 200 may operate using tracking information and other information provided by multiple XR headsets 150 such as inertial tracking information and optical tracking information (frames of tracking data). The XR headsets 150 operate to display visual information and may play-out audio information to the wearer. This information can be from local sources (e.g., the surgical robot 100), imaging devices 420 **(****FIG. 4****),** remote sources (e.g., patient medical image database), and/or other electronic equipment. The camera tracking system 200 may track fiducials in 6 degrees-of-freedom (6 DOF) relative to three axes of a 3D coordinate system and rotational angles about each axis. The XR headsets 150 may also operate to track hand poses and gestures to enable gesture-based interactions with "virtual" buttons and interfaces displayed through the XR headsets 150, and can also interpret hand or finger pointing or gesturing as various defined commands. Additionally, the XR headsets 150 may have a 1-10x magnification digital color camera sensor called a digital loupe. In some embodiments, one or more of the XR headsets 150 are minimalistic XR headsets that display local or remote information but include fewer sensors and are therefore more lightweight.

An "outside-in" machine vision navigation bar 206 supports the tracking cameras 204 and may include a color camera. The machine vision navigation bar generally has a more stable view of the environment because it does not move as often or as quickly as the XR headsets 150 while positioned on wearers' heads. The patient reference element (or, DRB) 116 is generally rigidly attached to the patient 210 with stable pitch and roll relative to gravity. This local rigid patient reference 116 can serve as a common reference for reference frames relative to other tracked elements, such as a reference element 114 on the end effector 112, instrument reference element 170, and reference elements on the XR headsets 150.

In some embodiments, at the end of the end effector 112, instruments are connected to perform operations such as resection, reaming, and implant placement.

The surgical robot 100 may be positioned near or next to patient 210 as shown in **FIGS. 1-2****.** The robot 100 can be positioned at any suitable location near the patient 210 depending on the area of the patient 210 undergoing the surgical procedure. The camera tracking system 200 may be separate from the robot system 100 and positioned at the foot of patient 210. This location allows the tracking camera 200 to have a direct visual line of sight to the surgical area 208, e.g., the hip area **(****FIG. 2****).** In the configuration shown in **FIG. 1****,** the surgeon 120 may be positioned across from the robot 100, but is still able to manipulate the end effector 112 and the display 110. A surgical assistant 126 may be positioned across from the surgeon 120 again with access to both the end effector 112 and the display 110. If desired, the locations of the surgeon 120 and the assistant 126 may be reversed. An anesthesiologist 122, nurse, or scrub tech can operate equipment which may be connected to display information from the camera tracking system 200 on a display 34 **(****FIG. 1****).**

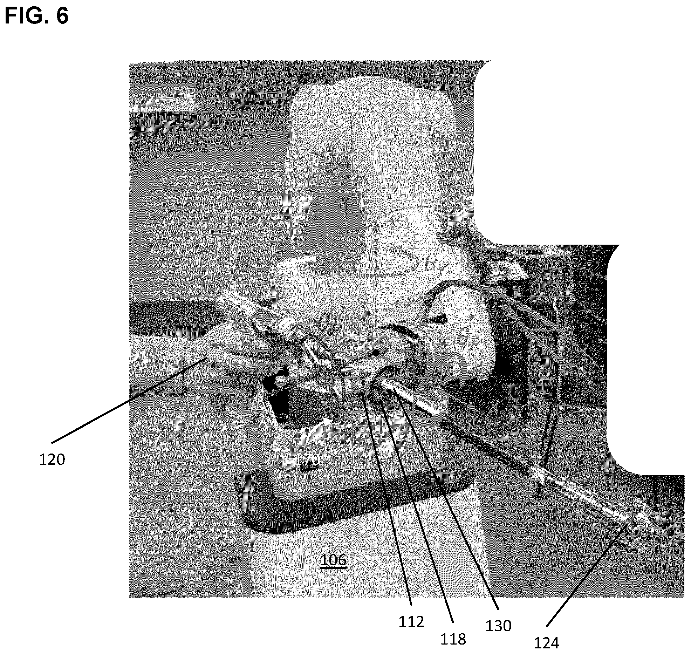

With respect to the other components of the robot 100, the display 110 can be attached to the surgical robot 100 or in a remote location. The end-effector 112 may be coupled to the robot arm 104 and be controlled by at least one motor. An upper arm 102 may further couple the arm 104 to the column 312 of the robot 100. In some embodiments, the end effector 112 includes a guide tube 118 (e.g., **FIG. 6****),** which is configured to receive and orient a surgical instrument, tool, or implant used to perform a surgical procedure on the patient 210. For example, the end effector 112 is adapted to receive (e.g. through a guide tube 118) a surgical instrument or a portion thereof, to removably couple to the instrument, and to manipulate the instrument such as by translating and rotating the instrument. In some other embodiments, the end-effector 112 includes a passive structure guiding a saw blade (e.g., sagittal saw) along a defined cutting plane.

As used herein, the term "end effector" is used interchangeably with the terms "end effectuator" and "effectuator element." The term "instrument" is used in a nonlimiting manner and can be used interchangeably with "tool" and "implant" to generally refer to any type of device that can be used during a surgical procedure in accordance with embodiments disclosed herein. The more general term, device, can also refer to structure of the end effector, etc. Example instruments, tools, and implants include, without limitation, reamer constructs, drills, screwdrivers, saws, dilators, retractors, probes, implant inserters, and implant devices such as shells and trial shells, screws, spacers, interbody fusion devices, plates, rods, etc. Although generally shown with a guide tube 118, it will be appreciated that the end-effector 112 may be replaced with any suitable instrumentation for use in surgery. In some embodiments, end-effector 112 can comprise any known structure for effecting the movement of the surgical instrument in a desired manner.

The surgical robot 100 is operable to control the translation and orientation of the end-effector 112. The robot 100 may move the end-effector 112 under computer control along x-, y-, and z-axes, for example. The end-effector 112 can be configured for selective rotation about one or more of the x-, y-, and z-axes, and a Z Frame axis, such that one or more of the Euler Angles (e.g., roll, pitch, and/or yaw) associated with the end effector 112 can be selectively computer controlled. In some embodiments, selective control of the translation and orientation of end effector 112 and associated surgical instrument can permit performance of medical procedures with significantly improved accuracy compared to conventional robots that utilize, for example, a 6 DOF robot arm comprising only rotational axes. For example, the surgical robot 100 may be used to operate on patient 210, and robot arm 104 can be positioned above the body of patient 210, with end-effector 112 selectively angled relative to the z-axis toward the body of patient 210.

In some example embodiments, the XR headset(s) 150 can be controlled to dynamically display an updated graphical indication of the pose of the surgical instrument so that the user, e.g., surgeon 120, can be aware of the pose of the surgical instrument at all times during the procedure.

In some further embodiments, surgical robot 100 can be operable to correct the path of a surgical instrument guided by the robot arm 104 if the surgical instrument strays from the selected, preplanned, or defined trajectory. The surgical robot 100 can be operable to permit stoppage, modification, and/or manual control of the movement of end effector 112 and/or the surgical instrument. Thus, in use, a surgeon 120 or other user can use the surgical robot 100 as part of computer assisted navigated surgery, and has the option to stop, modify, or manually control the autonomous or semi-autonomous movement of the end-effector 112 and/or the surgical instrument.

Fiducials of reference elements can be formed on or connected to robot arms 102 and/or 104, the end effector 112 (e.g., end effector element 114 in **FIG. 2****),** and/or a surgical instrument (e.g., instrument element 170) to enable tracking of poses in a defined coordinate system, e.g., such as in six degrees of freedom (DOF) along three orthogonal axes and rotation about the axes. The reference elements 114, 116, 170 enable each of the marked objects (e.g., the end-effector 112, the patient 210, and the surgical instruments, respectively) to be tracked by the tracking camera 200, and the tracked poses can be used to provide navigated guidance during a surgical procedure and/or to control movement of the surgical robot 100 for guiding the end effector 112 and/or an instrument manipulated by the end effector 112. The instrument manipulated by the end effector 112 may include, e.g., a reamer 124 or an inserter adapted to insert an implant.

Referring to **FIG. 3** the surgical robot 100 may include a display 110, upper arm 102, lower arm 104, end effector 112, vertical column 312, casters 314, a table 318, and ring 324 which uses lights to indicate statuses and other information. Cabinet 106 may house electrical components of surgical robot 100 including, but not limited to, a battery, a power distribution module, a platform interface board module, and a computer. The camera tracking system 200 may include a display 36, tracking cameras 204, arm(s) 202 **(****FIG. 1****),** a computer housed in cabinet 330, and other components.

In computer assisted navigated surgeries, perpendicular 2D scan slices, such as axial, sagittal, and/or coronal views of patient anatomical structure are displayed to enable user visualization of the patient's anatomy alongside the relative poses of surgical instruments. An XR headset or other display can be controlled to display one or more 2D scan slices of patient anatomy along with a 3D graphical model of anatomy. The 3D graphical model may be generated from a 3D scan of the patient, e.g., by a CT scan device, and/or may be generated based on a baseline model of anatomy which isn't necessarily formed from a scan of the patient.

### Example Surgical System

**FIG. 4** illustrates a block diagram of a surgical system 10 that includes a surgical robot 100, a computer platform 400 including, *inter alia,* the camera tracking system 200, imaging device(s) 420, and XR headset(s) 150 which are configured to operate as described herein, according to some embodiments.

The imaging device(s) 420 may include a C-arm imaging device, an O-arm imaging device, other imaging device, and/or a patient image database of 2D and/or 3D images. The XR headset 150 provides a human interface for performing navigated surgical procedures. The XR headset 150 can be configured to provide functionalities, e.g., via the computer platform 400, that include without limitation any one or more of: identification of hand gesture-based commands, and display of XR graphical objects on a display device 438 of the XR headset 150 and/or another display device. The display device 438 may include a video projector, flat panel display, etc. The user may view the XR graphical objects as an overlay anchored to particular real-world objects viewed through a see-through display screen. The XR headset 150 may additionally or alternatively be configured to display on the display device 438 video streams from cameras mounted to one or more XR headsets 150 and other cameras.

Electrical components of the XR headset 150 can include a plurality of cameras 430, a microphone 432, a gesture sensor 434, a pose sensor (e.g., inertial measurement unit (IMU)) 436, the display device 438, and a wireless/wired communication interface 440. The cameras 430 of the XR headset 150 may be visible light capturing cameras, near infrared capturing cameras, or a combination of both.

The cameras 430 may be configured to operate as the gesture sensor 434 by tracking for identification user hand gestures performed within the field-of-view of the camera(s) 430. Alternatively, the gesture sensor 434 may be a proximity sensor and/or a touch sensor that senses hand gestures performed proximately to the gesture sensor 434 and/or senses physical contact, e.g., tapping on the sensor 434 or its enclosure. The pose sensor 436, e.g., IMU, may include a multi-axis accelerometer, a tilt sensor, and/or another sensor that can sense rotation and/or acceleration of the XR headset 150 along one or more defined coordinate axes. Some or all of these electrical components may be contained in a head-worn component enclosure or may be contained in another enclosure configured to be worn elsewhere, such as on the hip or shoulder.

As explained above, the surgical system 10 includes the camera tracking system 200 which may be connected to a computer platform 400 for operational processing and which may provide other operational functionality including a navigation controller 404 and/or an XR headset controller 410. The surgical system 10 may further include the surgical robot 100. The navigation controller 404 can be configured to provide visual navigation guidance to an operator for moving and positioning a surgical tool relative to patient anatomical structure based on a surgical plan, e.g., from a surgical planning function, defining where a surgical procedure is to be performed using the surgical tool on the anatomical structure and based on a pose of the anatomical structure determined by the camera tracking system 200. The navigation controller 404 may be further configured to generate navigation information based on a target pose for a surgical tool, a pose of the anatomical structure, and a pose of the surgical tool and/or an end effector 112 of the surgical robot 100. The navigation information may be displayed through the display device 438 of the XR headset 150 and/or another display device to indicate where the surgical tool and/or the end effector 112 of the surgical robot 100 should be moved to perform a surgical procedure according to a defined surgical plan.

The electrical components of the XR headset 150 can be operatively connected to the electrical components of the computer platform 400 through the wired/wireless interface 440. The electrical components of the XR headset 150 may be operatively connected, e.g., through the computer platform 400 or directly connected, to various imaging devices 420, e.g., the C-arm imaging device, the O-arm imaging device, other imaging device(s), the patient image database, and/or to other medical equipment through the wired/wireless interface 440.

The surgical system 10 may include a XR headset controller 410 that at least partially resides in the XR headset 150, the computer platform 400, and/or another system component connected via wired cables and/or wireless communication links. Various functionality may be provided by software executed by the XR headset controller 410. The XR headset controller 410 is configured to receive information from the camera tracking system 200 and the navigation controller 404, and to generate an XR image based on the information for display on the display device 438.

The XR headset controller 410 can be configured to operationally process frames of tracking data from the cameras 430 (tracking cameras), signals from the microphone 432, and/or information from the pose sensor 436 and the gesture sensor 434, to generate information for display as XR images on the display device 438 and/or for display on other display devices for user viewing. Thus, the XR headset controller 410 as illustrated as a circuit block within the XR headset 150 is to be understood as being operationally connected to other illustrated components of the XR headset 150 but not necessarily residing within a common housing or being otherwise transportable by the user. For example, the XR headset controller 410 may additionally or alternatively reside within the computer platform 400 which, in turn, may reside within the cabinet 330 of the camera tracking system 200, the cabinet 106 of the surgical robot 100, etc.

### Exemplary Patient Registration Workflows

In some embodiments of the present disclosure, the system 10, e.g., computer platform 400, may perform one of a number of available workflows to register a patient to the surgical system 10 prior to surgery. The workflows may further include isolating a target area for the surgical procedure from non-target surgical areas. In one example, the target surgical area may include the acetabulum, and the non-target surgical area may include the femur.

In one embodiment, the workflow may be an imageless workflow in which no pre-operative images are used. Instead, information about the patient anatomy in the operating room (OR) can be obtained by the surgeon measuring key parameters of the patient's bone using the system as described herein. For example, the computer platform 400 of the system 10 operates to identify the locations of landmarks (e.g., points, axes, and/or surfaces) on the bone and register the locations either concurrently with the identification or thereafter. The locations can be used to define reference plane(s) (e.g., anterior pelvic plane (APP) and/or functional pelvic plane (FPP)) which, in turn, are used to plan implants and navigate the robot and surgical instruments for THA surgical procedures.

In some embodiments, the only pre-operative use case associated with the imageless workflow may be the initial patient assessment. The surgeon may assess the patient's mobility and health status with assistance from sensors (e.g., sensors made by Globus Medical which are attached to the leg), physical exercises, and/or clinical surveys to determine if THA is recommended. Gathered data may then be stored and processed by the system before being analyzed by the surgeon to facilitate a final decision. Subsequently, the data may be reused by an application (e.g., surgery planning application by Globus Medical) to establish the most appropriate implant surgical plan.

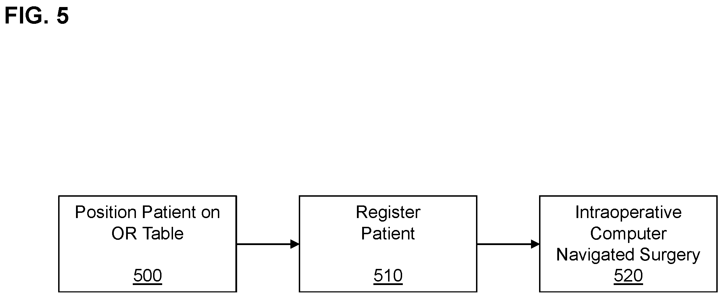

**FIG. 5** illustrates a flowchart for a workflow during an intra-operative portion of a THA surgery, in accordance with some embodiments of the present disclosure. In some embodiments, after positioning the patient on the operating room table (process 500), some of the operations discussed above and below may be performed during process 510 to register a patient and before another process 520 for intraoperative computer navigated surgery. In the case of a hip, a pelvis or acetabulum of the patient is registered in the tracking coordinate system of the camera tracking system 200. As shown in **FIG. 1****,** the pelvis or acetabulum is registered in the optical coordinate system. In one embodiment, the registration is done in an imageless modality without the use of any medical images such as X-rays or CT images from an imaging device. As noted herein, in other embodiments, registration is performed using one or more pre-operative X-ray images and/or CT images.

**FIG. 6** illustrates a configuration of end effector 112 used in a force control mode according to certain implementations. Aspects of the force control mode, as well as additional modes (e.g., point rotate control mode, axis rotate control mode, translate control mode, translate/rotate control mode, etc.) are further described in US Patent Application No. 18/737,123 (Docket ROBOT.145.0002), previously incorporated by reference herein.

### Exemplary End Effectors with Instrument Interface

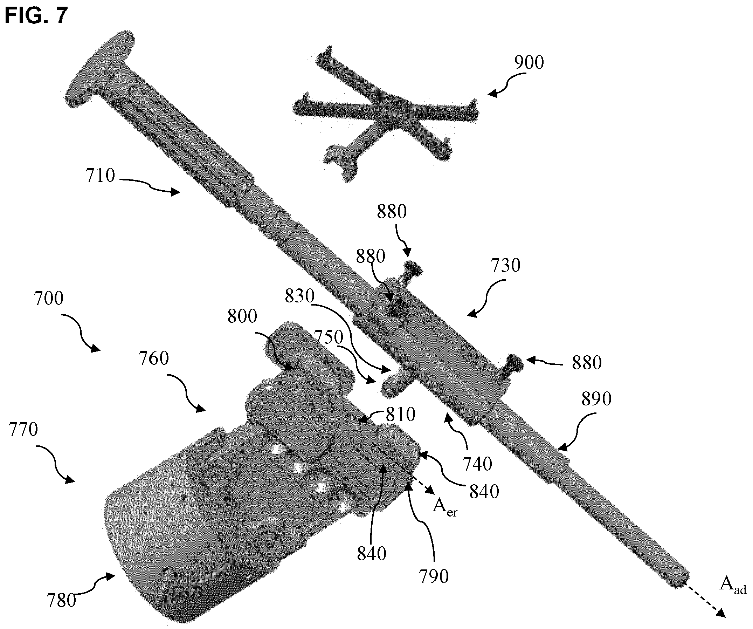

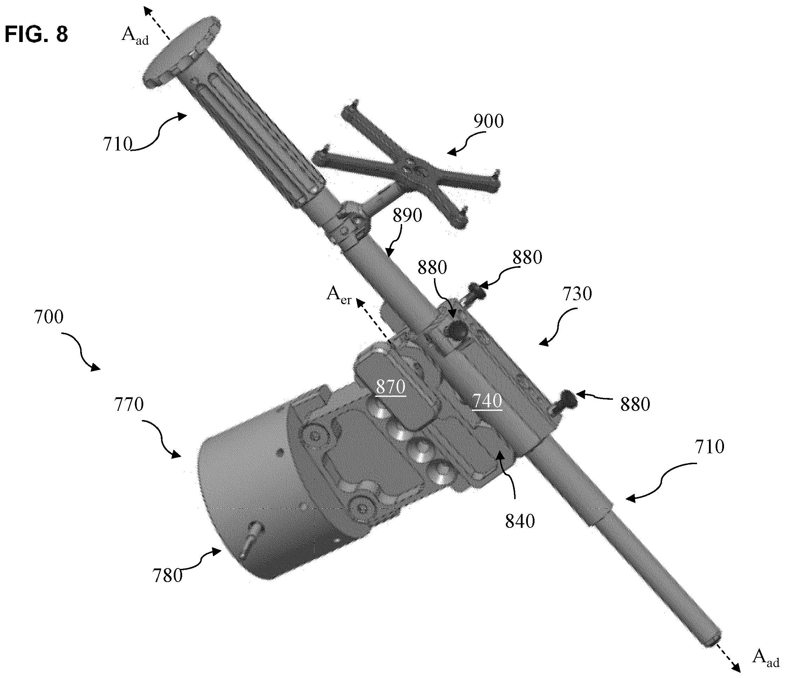

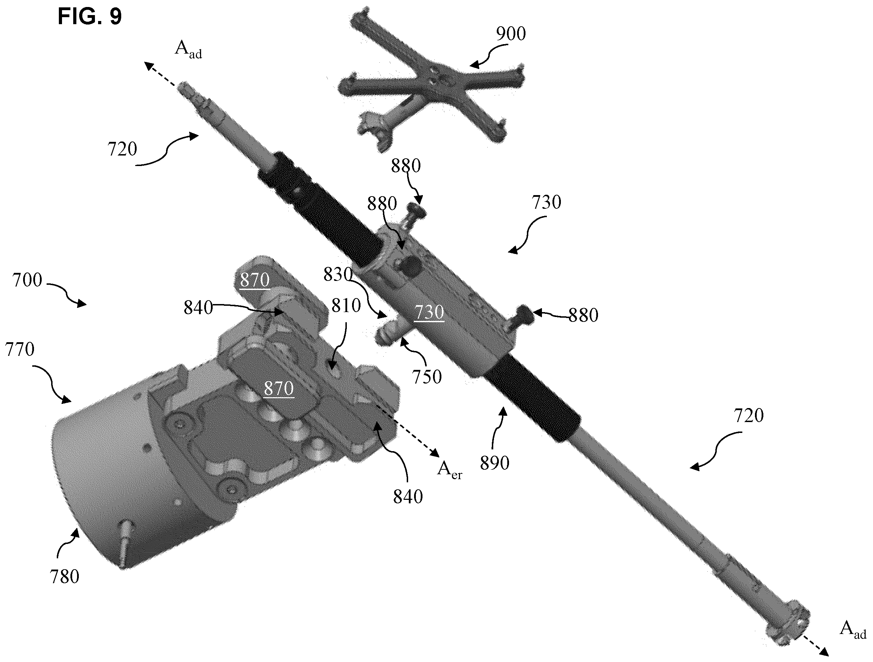

**FIG. 7** shows a perspective separated (blow-out or exploded) view of an example end effector 700 interfacing with a surgical instrument 710 according to various implementations. **FIG. 8** shows components of **FIG. 7** in assembled form. **FIG. 9** shows the end effector 700 interfacing with a distinct surgical instrument 720. As described herein, the end effector 700 can be configured to interface with a plurality of types of surgical instrument, e.g., reamers, impactors, etc., and enable efficient connection and disconnection of such instruments to beneficially enhance surgical procedures such as a total hip arthroplasty (THA) procedure.

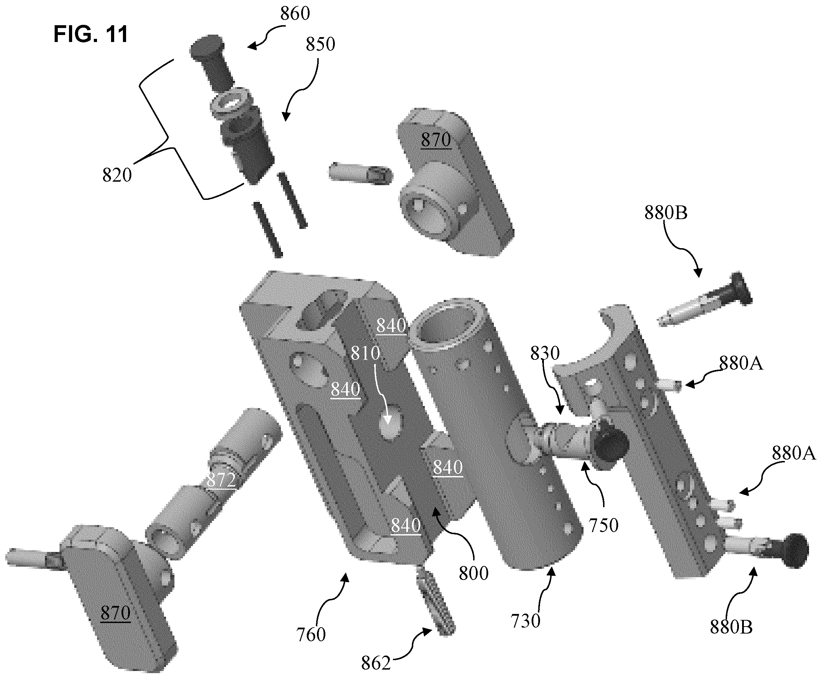

With reference to **FIGS. 7-9****,** the end effector 700 is shown including an adapter 730 for holding the surgical instrument 710, 720. In particular implementations, the adapter 730 has an elongate body 740, with a projection 750 extending therefrom. The end effector 700 further includes a main body 760 that has a proximal end 770 including an interface 780 for attaching to a robot arm 104 (FIG. 2). The main body 760 also has a distal end 790 that includes an elongate recess 800 and a slot 810 for connecting with the surgical instrument 710, 720, e.g., by receiving the projection 750 extending therefrom. In various implementations, a connector 820 is adapted to secure the projection 750 in the slot 810 to securely attach the adapter 730 to the main body 760, as shown in the disassembled perspective view of a portion of the end effector 700 in **FIG. 11****.**

According to certain embodiments, the projection 750 complements the slot 810 in the main body 760, e.g., a cross sectional shape of the projection 750 corresponds to and mates with the cross sectional shape of the slot 810. In various implementations, the projection 750 extends approximately perpendicularly from the primary axis (A_{ad}) of the adapter 730, and is configured to align with slot 810 that extends approximately perpendicularly from the primary axis (Aₑᵣ) of the elongate recess 800.

In certain cases, the projection 750 includes a notch 830 that complements the connector 820 and enables attachment and detachment of the adapter 730 from the main body 760 as explained herein below. In some examples, the notch 830 includes a V-shaped slot. Other shapes are also possible, e.g., a U-shaped slot, an arcuate slot, or an oblong shaped slot. In particular cases, when secured to the end effector 700 (e.g., as shown in **FIG. 8****),** the adapter 730 interfaces with the elongate recess 800, and is coupled with the connector 820 **(****FIG. 11****).**

The elongate recess 800 can take various forms, and in particular cases, is at least partially defined by sidewalls 840 extending in a distal direction from the distal end 790 of the main body 760. In certain cases, the sidewalls 840 extend along at least a portion of the length of the main body 760. In some examples, the sidewalls 840 extend along only a portion of the length of the main body 760 along Aₑᵣ. In further examples, the sidewalls 840 include distinct protrusions or sections extending from the main body 760. Sidewalls 840 can define various shapes of elongate recess 800, including, for example, a V-shaped cross-section, a U-shaped cross-section, or a polygonal shaped cross-section (as shown in **FIG. 11****).**

In certain implementations, the shape and dimensions of the adapter 730 complement the shape and dimensions of the elongate recess 800. In a particular example, the adapter 730 is cylindrical and complements the elongate recess 800 (e.g., a V-shaped or U-shaped recess). According to various implementations, the sidewalls 840 are spaced to accommodate the outer dimension, e.g., diameter of the cylindrical (or other geometry) adapter 730, e.g., such that the adapter 730 contacts the sidewalls 840 and the distal end 790 of the main body 760 when the adapter 730 is secured.

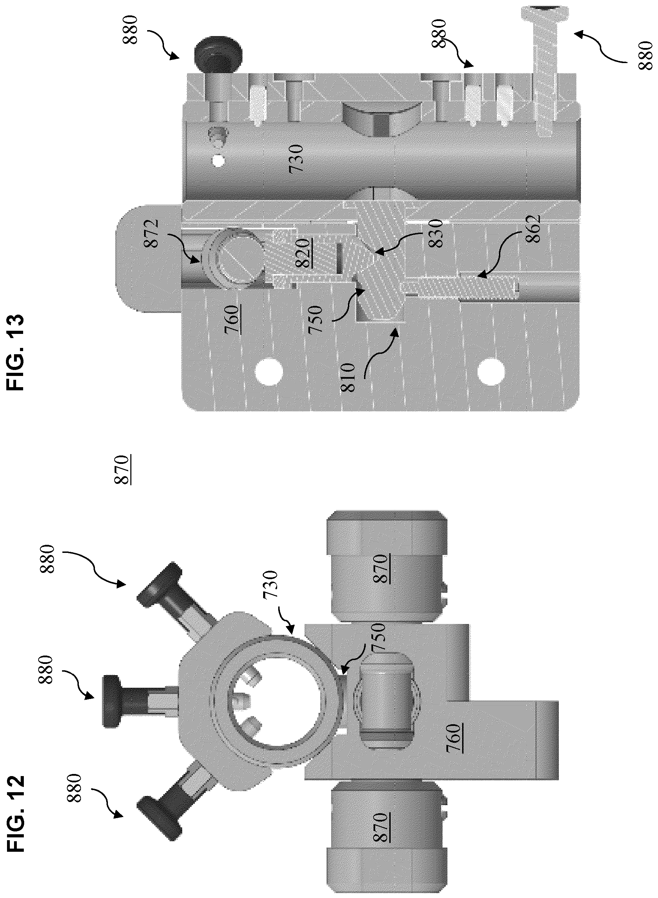

In various implementations, as shown in **FIG. 11****,** along with the end view of a portion of end effector 700 in **FIG. 12** and the cross-sectional view of a portion of end effector 700 in **FIG. 13****,** the connector 820 can include a pin 850 sized for insertion or removal from the notch 830 in the projection 750 in the adapter 730. In some cases, the pin 850 includes a notch, a tab, or a recess that is configured to engage the notch 830. In particular cases, the connector 820 includes a spring-mounted coupling 860. In certain aspects, the pin 850 can be actuated by an actuator 870 such as a handle, tab, or switch, that is configured to extend/retract the pin 850, or swing or rotate the pin 850 between positions to engage/disengage the notch 830. According to certain embodiments, the actuator 870 enables a user to couple or decouple the surgical instrument 710, 720 from the main body 760. In some cases, the actuator 870 has a multi-modal actuation mechanism, e.g., for engaging and/or disengaging the pin 850 from the notch. For example, the actuator 870 may require a compress-and-turn actuation, a slide-and-turn actuation, or a slide-and-compress actuation to engage or disengage the pin 850 from the notch. In some cases, the actuator 870 can be actuated with a single operator's hand. In particular examples, the actuator 870 includes two linked handles **(****FIG. 11****)** that are joined by a central shaft 872, and enable actuation of the pin 850 from multiple sides of the end effector 700. The central shaft 872 rotates around a central axis and has a center portion of reduced diameter and which is off axis to act as a cam to push the pin 850 down (by pushing on coupling 860) when the knob 870 is rotated. In additional implementations, a secondary coupler 862, e.g., a pin or screw, can be positioned in a slot within the main body to interface with the projection 750 on a side that opposes the notch 830. In some cases, the secondary coupler 862 is fixed in the main body 760 and protrudes slightly into the slot 810, e.g., enabling the projection 750 to slide past the coupler 862 into the slot 810 when the connector 820 is not engaged with the notch 830.

According to certain embodiments, the surgical instrument 710, 720 and the adapter 730 are configured to connect with the main body 760 as a single unit. For example, in certain implementations, the surgical instrument 710, 720 and adapter 730 can be pre-coupled and connected with the main body 760 using a quick-connect approach, e.g., with a single operator hand. For example, the surgical instrument 710, 720 and adapter 730 can be pre-coupled by the operator (e.g., surgeon) or a surgical assistant, and the operator can then connect the adapter 730 to the main body 760 using a single hand. This can enable the operator to effectively connect the adapter 730 to the main body 760 while engaged in other surgical functions, e.g., with another hand. In certain of these cases, a final connection is made with a second operator's hand, e.g., the operator and/or a surgical assistant.

In particular implementations, as described herein, the connector 820 and the elongate recess 800 limit at least two degrees of freedom (DoF) of the surgical instrument 710, 720. According to certain embodiments, the adapter 730 includes at least two limiters 880 to limit degrees of freedom (DoF) of the surgical instrument 710, 720. For example, limiters 880 can be configured to limit translation and rotation of the surgical instrument 710, 720. In some examples, limiters 880 can include buttons, protrusions, or other interfaces. In one example implementation shown in **FIGS. 11-13****,** limiters 880 include pins 880A and/or screws 880B that are configured to interface with the outer surface 890 **(****FIGS. 7-10****)** of the surgical instrument 710, 720 and/or interface with slots, grooves, or apertures in the surgical instrument 710, 720 (not shown). In further implementations, when secured, the degrees of freedom (DoF) of the surgical instrument 710, 720 relative to the end effector 700 is approximately zero.

In particular cases, the connector 820 enables decoupling of the surgical instrument 710, 720 (e.g., impactor) from the main body 760 in a single disconnect process, restoring at least two DoF limited by the end effector 700. For example, in some cases, the operator (e.g., surgeon) may beneficially perform the single disconnect process to test the grip and/or fit of an implant in an implant pocket. In these examples, the operator may wish to manipulate the surgical instrument without constraint by the end effector 700, e.g., free of the surgical robot arm 104. This grip and/or fit test is frequently performed by hand, and enabling efficient connection and/or disconnection from the end effector 700 can enhance the effectiveness and efficiency of the THA procedure.

In additional cases, the operator may beneficially perform the single disconnect process if the surgical robot 100 fails or locks for any reason. For example, the surgical robot 100 can arrest or seize for one or more reasons, limiting movement of the surgical instrument 710, 720 due to a power supply interruption, software malfunction, and/or a surgical process limitation (e.g., induced by a controller and/or related software). In such cases, the surgeon may wish to manually manipulate the surgical instrument 710, 720 to complete one or more portions of a procedure (e.g., a THA procedure). That is, in many cases, decoupling the surgical instrument 710, 720 (e.g., impactor) from the main body 760 enables manual manipulation of the surgical instrument 710, 720 by the surgeon. Further, in various implementations, surgical instrument 710, 720 is configured to be coupled or decoupled from the main body 760 at any stage of the THA procedure, e.g., during reaming, impacting, positioning, etc.

As noted herein, the adapter 730 can be configured to interface (e.g., couple) with a variety of surgical instruments, e.g., impactors, reamers, placement devices, etc., that can be used in performing a THA procedure. In some aspects, the adapter 730 can couple with a plurality of surgical instruments during a THA procedure. In additional implementations, a surgical procedure can be aided by having a plurality of adapters 730 available to pre-couple to a variety of distinct surgical instruments 710, 720, simplifying the process of connecting and disconnecting those surgical instruments 710, 720 from the main body 760.

According to certain embodiments, as illustrated in **FIGS. 7-9****,** the surgical instrument 710, 720 includes a navigation array 900. As described herein, the navigation array 900 can enable precise tracking of a position of the instrument 710, 720 during the surgical procedure, e.g., THA procedure. The navigation array 900 can enable optical tracking of the position of the instrument 710, 720 throughout the THA procedure, enhancing control of the surgical robot 100 and improving surgical outcomes. In particular implementations, distinct types of navigation array 900 are coupled with distinct types of surgical instrument 710, 720, such that a first navigation array 900 is paired with a surgical instrument 710 (e.g., impactor) and a second navigation array 900 is paired with a second surgical instrument 720 (e.g., reamer). For example, a navigation array 900 paired with a reamer may be configured not to move with rotation of the reamer, while a navigation array 900 paired with an impactor may be configured to move with rotation of the impactor.

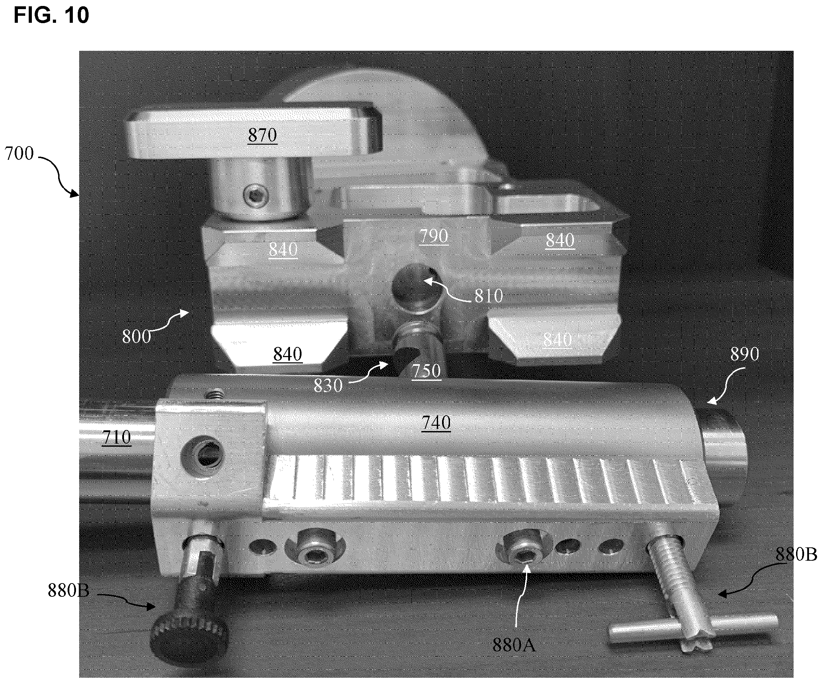

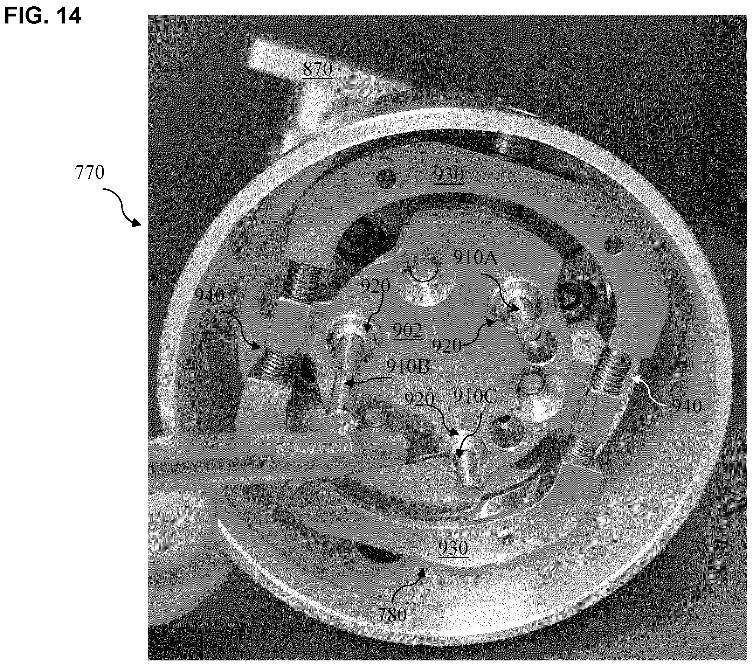

Turning to **FIGS. 10-14****,** with particular reference to **FIG. 14****,** the interface 780 on the proximal end 770 of the main body 760 can include a plate 902 with a set of pins 910 extending therefrom for interfacing with the robot arm 104. In one example, the set of pins 910 includes two pins, three pins, four pins, or more. In a particular example, the set of pins 910 includes three pins 910A, 910B, 910C. In various implementations, the set of pins 910 are used as alignment pins for aligning with apertures in an interface plate on the robot arm 104. In certain implementations, the end effector 700 further includes kinematic mounts 920 proximate each of the pins 910. In some cases, the interface 780 includes a set of two U-shaped elements 930 adjustably coupled by at least one screw 940 (two shown). In certain cases, the U-shaped elements 930 surround the plate 902. The screws 940 can enable relative adjustment of the U-shaped elements 930 to enhance a clamping force on the connection with the robot arm 104. For example, the screws 940 can be tightened to clamp the U-shaped elements 930 around a coupler on the robot arm 104.

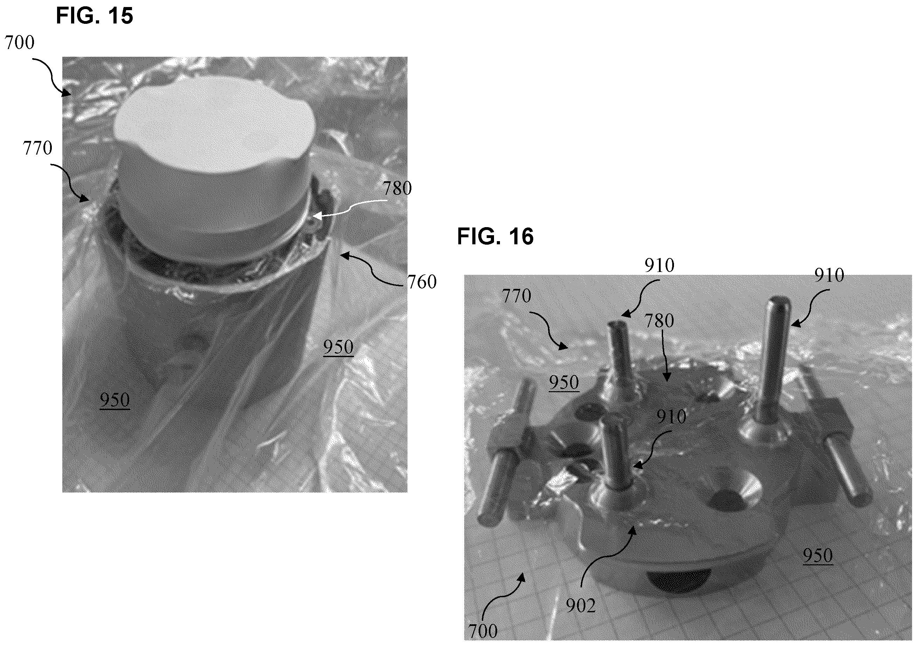

According to certain embodiments, as illustrated in **FIGS. 15 and 16****,** the end effector 700 can further include a drape 950 across the interface 780 on the proximal end 770 of the main body 760. In particular cases, the drape 950 provides a sterile shield between the actuator 870 and the surgical instrument 710, 720, e.g., during the THA procedure. According to certain embodiments, the sterile shield or drape 950 allows the user to couple or decouple the surgical instrument 710, 720 from the main body 760 without compromising pre-established sterility.

According to certain embodiments, a method of operating the end effector 700 includes coupling the end effector 700 to the surgical instrument 710, 720, e.g., via the adapter 730. As described herein, the surgical instrument 710, 720 can be decoupled from the end effector 700 without repositioning the robot arm 104. For example, decoupling the end effector 700 can be performed without moving the arm 104 out of the surgical field.

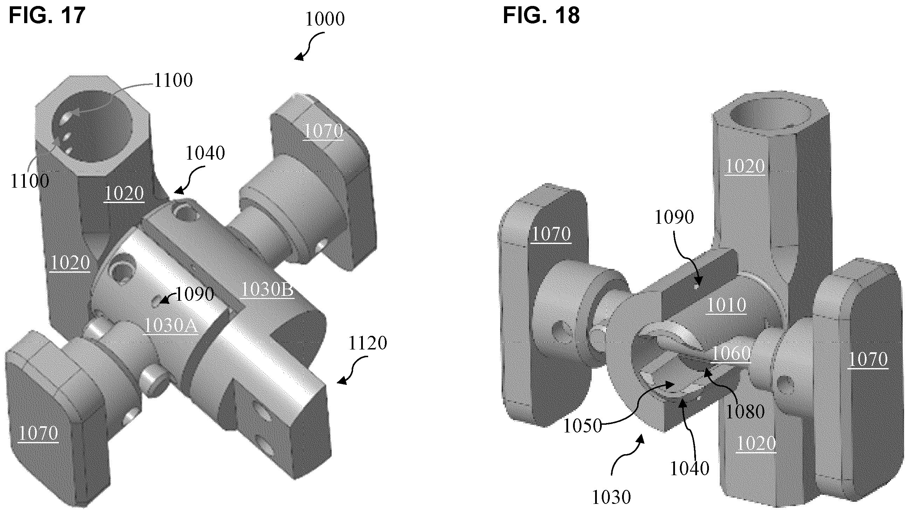

In addition to the end effector 700 shown and described according to various implementations, other implementations can include an end effector 1000 with a distinct connection mechanism for coupling with an adapter 1020. As shown in **FIGS. 17 and 18****,** adapter 1020 can be configured to receive a surgical instrument (e.g., surgical instrument 710, 720), and interface with the main body 1030 of an end effector 1000 (which in some implementations includes multiple sections). The adapter 1020 can include a protrusion 1010 that is sized to complement a slot 1040 in the main body 1030, and can be coupled with the main body 1030 using a quick connect approach as described herein. In particular cases, the protrusion 1010 includes an internal slot 1050 that is configured to interface with a shaft (or, pin) 1060 in the main body 1030. The shaft (or, pin) 1060 can extend between handles 1070 (e.g., the handles 1070 may be connected by shaft 1060), and can further include a retention member 1080 that can be rotated, slid, or otherwise actuated into place to engage a corresponding retention feature on the protrusion 1010. In certain aspects, the main body 1030 is formed from distinct components 1030A, 1030B that are connected via couplers 1090 such as pins or screws. In various implementations, the adapter 1020 is configured to couple with any of a plurality of distinct types of surgical instrument 710, 720, and similar to adapter 730, can include apertures (or slots) 1100 for receiving a retention member such as a screw or pin to retain the surgical instrument. Portions of the end effector 1000 are shown in **FIG. 17** and **FIG. 18****,** and the end effector 1000 can include a coupler 1120 that interfaces with the proximal end 770 of the main body 760 **(****FIG. 7****).** Similar to end effector 700, end effector 1000 is configured to limit the DoF of the surgical instrument 710, 720 to enhance surgical outcomes. Further, similar to end effector 700, end effector 1000 can be configured to enable a quick connect approach with the robot arm 104, and further enables the operator to disconnect the adapter 1020 to restore the DoF of the surgical instrument 710, 720 while the robot arm 104 remains in the surgical field.

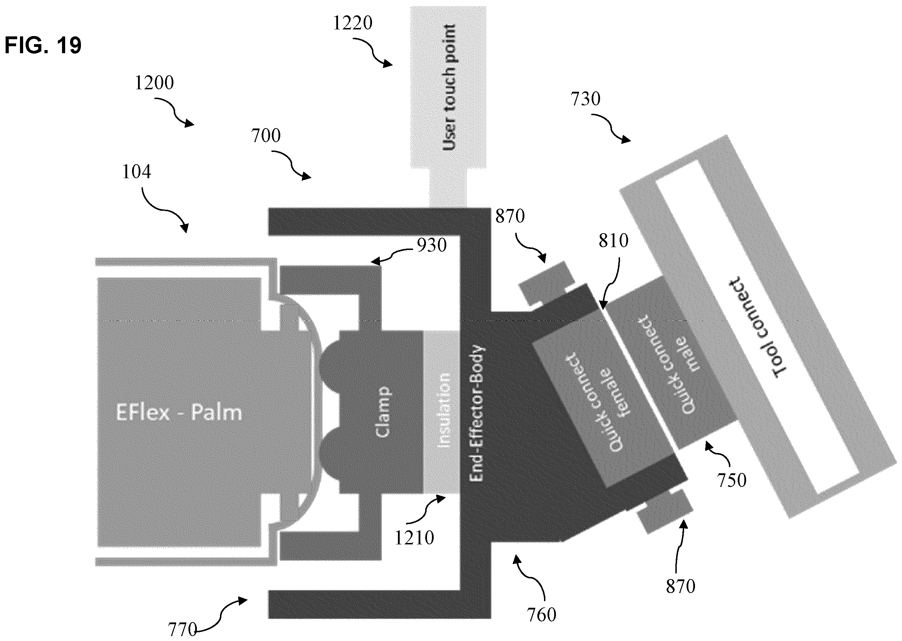

**FIG. 19** shows a schematic depiction of an example system 1200 that includes the robot arm 104 (e.g., also referred to as an EFlex-Palm) coupled with an end effector 700 according to various implementations. It is understood that the end effector 700 in this example system 1200 can be substituted with end effector 1000 depicted in **FIG. 15** and **FIG. 16** and/or any other end effector shown and described herein. The end effector 700 is illustrated in an angled position relative to the robot arm 104 in **FIG. 19****,** but it is understood that the end effector 700 could also be aligned with the robot arm 104 such the main body 760 is not angled.

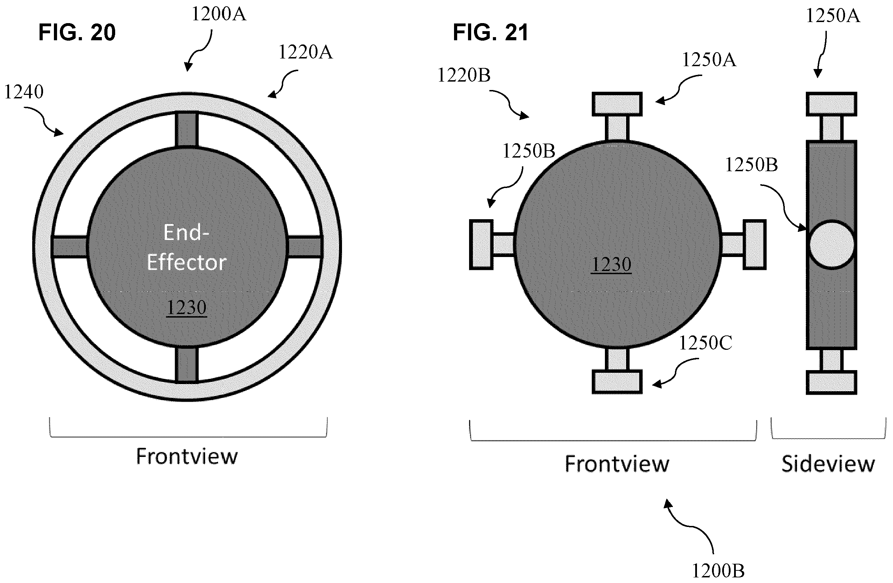

In addition to components of the end effector 700 illustrated in **FIGS. 6-18****,** system 1200 also illustrates an optional insulation layer 1210 within the main body 760. Also shown is a user touch point 1220, which can enable a user (e.g., surgeon or other operator) to interface with the end effector 700. Optional configurations for user touch points 1220A, 1220B are shown in **FIGS. 20 and 21****,** respectively. These user touch points 1200 can include a knob 1230 with a continuous grip element 1240 or a multi-pronged grip 1250 defined by distinct prongs 1250A, 1250B, etc. In certain cases, the user touch points 1200 enable adjustment of the end effector 700, and in some cases, enable coupling and/or decoupling of the end effector 700 from the robot arm 104. In some aspects, actuation of the user touch points 1200 controls the clamping of U-shaped elements 930 to connect/disconnect from the robot arm 104. These touch points 1200 can be actuated with a single user hand in certain implementations.

The end effectors illustrated and described according to various implementations can provide benefits relative to conventional end effectors and surgical instruments. For example, the end effectors disclosed according to various implementations can be configured to interface with a plurality of types of surgical instrument, e.g., reamers, impactors, placement devices, etc., and enable efficient connection and disconnection of such instruments to beneficially enhance surgical procedures such as a total hip arthroplasty (THA) procedure. The end effectors disclosed herein can further enable an operator to efficiently disconnect a surgical instrument from a robot arm in the surgical field, enhancing aspects of a THA procedure such as reaming, impacting, and/or implant placement.

### Further Definitions and Embodiments

In the above description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected," "coupled," "responsive," or variants thereof to another element, it can be directly connected, coupled, or responsive to the other element or intervening elements may be present. In contrast, when an element is referred to as being "directly connected," "directly coupled," "directly responsive," or variants thereof to another element, there are no intervening elements present. Like numbers refer to like elements throughout. Furthermore, "coupled," "connected," "responsive," or variants thereof as used herein may include wirelessly coupled, connected, or responsive. As used herein, the singular forms "a," "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise," "comprising," "comprises," "include," "including," "includes," "have," "has," "having," or variants thereof are openended, and include one or more stated features, integers, elements, steps, components or functions but does not preclude the presence or addition of one or more other features, integers, elements, steps, components, functions or groups thereof. Furthermore, as used herein, the common abbreviation "e.g.," which derives from the Latin phrase "exempli gratia," may be used to introduce or specify a general example or examples of a previously mentioned item, and is not intended to be limiting of such item. The common abbreviation "i.e.," which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the spirit and scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

The invention could be defined inter alia by the following examples:
1. An end effector for a total hip arthroplasty (THA) procedure, the end effector comprising: an adapter for holding a surgical instrument, the adapter having an elongate body and a projection extending therefrom; a main body having: a proximal end including an interface for attaching to a robot arm; and a distal end including an elongate recess and a slot for connecting with the surgical instrument; and a connector adapted to secure the projection in the slot to securely attach the adapter to the main body.
2. The end effector of claim 1, wherein the connector and the elongate recess limit at least two degrees of freedom (DoF) of the surgical instrument.
3. The end effector of example 1, wherein the surgical instrument and the adapter are configured to connect with the main body as a single unit.
4. The end effector of example 1, wherein the projection complements the slot in the main body.
5. The end effector of example 4, wherein the projection includes a notch that complements the connector and enables attachment and detachment of the adapter from the main body.
6. The end effector of example 1, wherein when secured, the adapter interfaces with the elongate recess and is coupled with the connector.
7. The end effector of example 1, wherein the adapter includes at least two limiters to limit degrees of freedom (DoF) of the surgical instrument.
8. The end effector of example 1, wherein the surgical instrument is of a first type of a plurality of types of surgical instrument configured to detachably couple with the main body.
9. The end effector of example 1, wherein the surgical instrument includes an impactor.
10. The end effector of example 9, wherein the connector enables decoupling of the impactor from the main body in a single disconnect process, restoring at least two DoF limited by the end effector.
11. The end effector of example 10, wherein decoupling the impactor from the main body enables manual manipulation of the impactor by a surgeon.
12. The end effector of example 1, wherein the surgical instrument is configured to be coupled or decoupled from the main body at any stage of the THA procedure.
14. The end effector of example 1, wherein the surgical instrument includes a navigation array.
15. The end effector of example 1, wherein the elongate recess has one of a V-shaped cross-section or a U-shaped cross-section.
16. The end effector of example 15, wherein the adapter is cylindrical and complements the elongate recess.
17. The end effector of example 1, wherein the connector includes a pin sized for insertion or removal from a slot in the projection in the adapter.
18. The end effector of example 1, wherein when secured, degrees of freedom (DoF) of the surgical instrument relative to the end effector is approximately zero.
19. The end effector of example 1, wherein the interface on the proximal end includes a set of pins for interfacing with the robot arm.
20. The end effector of example 19, further comprising kinematic mounts proximate each of the pins.
21. The end effector of example 19, wherein the interface includes a set of two U-shaped elements adjustably coupled by at least one screw.
22. The end effector of example 1, wherein the connector includes an actuator enabling a user to couple or decouple the surgical instrument from the main body.
23. The end effector of example 22, further comprising a drape across the interface on the proximal end of the main body, wherein the drape provides a sterile shield between the actuator and the surgical instrument.
24. The end effector of example 23, wherein the sterile shield allows the user to couple or decouple the surgical instrument from the main body without compromising pre-established sterility.
25. A method of coupling the end effector of example 1 to the surgical instrument.
26. The method of example 25, further comprising decoupling the end effector from the surgical instrument without repositioning the robot arm.
27. A surgical robot comprising the robot arm coupled with the end effector of example 1.

## Claims

1. An end effector for a total hip arthroplasty (THA) procedure, the end effector comprising:
an adapter for holding a surgical instrument, the adapter having an elongate body and a projection extending therefrom;
a main body having:
a proximal end including an interface for attaching to a robot arm; and
a distal end including an elongate recess and a slot for connecting with the surgical instrument; and
a connector adapted to secure the projection in the slot to securely attach the adapter to the main body.

2. The end effector of claim 1, wherein the connector and the elongate recess limit at least two degrees of freedom (DoF) of the surgical instrument.

3. The end effector of claim 1 or 2 , wherein the surgical instrument and the adapter are configured to connect with the main body as a single unit.

4. The end effector of any one of the preceding claims, wherein the projection complements the slot in the main body, and wherein preferably the projection includes a notch that complements the connector and enables attachment and detachment of the adapter from the main body.

5. The end effector of any one of the preceding claims, wherein when secured, the adapter interfaces with the elongate recess and is coupled with the connector.

6. The end effector of any one of the preceding claims, wherein the adapter includes at least two limiters to limit degrees of freedom (DoF) of the surgical instrument.

7. The end effector of any one of the preceding claims, wherein the surgical instrument is of a first type of a plurality of types of surgical instrument configured to detachably couple with the main body and wherein preferably the surgical instrument includes an impactor and wherein preferably the connector is so configured to enable decoupling of the impactor from the main body in a single disconnect process, restoring at least two DoF limited by the end effector.

8. The end effector of claim 7, wherein decoupling the impactor from the main body enables manual manipulation of the impactor by a surgeon.

9. The end effector of any one of the preceding claims, wherein the surgical instrument is configured to be coupled or decoupled from the main body at any stage of the THA procedure and wherein preferably the surgical instrument includes a navigation array.

10. The end effector of any one of the preceding claims, wherein the elongate recess has one of a V-shaped cross-section or a U-shaped cross-section and wherein preferably the adapter is cylindrical and complements the elongate recess.

11. The end effector of claim 1, wherein the connector includes a pin sized for insertion or removal from a slot in the projection in the adapter and wherein preferably when secured, degrees of freedom (DoF) of the surgical instrument relative to the end effector is approximately zero.

12. The end effector of any one of the preceding claims, wherein the interface on the proximal end includes a set of pins for interfacing with the robot arm and further preferably comprising kinematic mounts proximate each of the pins and wherein preferably the interface includes a set of two U-shaped elements adjustably coupled by at least one screw.

13. The end effector of any one of the preceding claims, wherein the connector includes an actuator enabling a user to couple or decouple the surgical instrument from the main body.

14. The end effector of claim 13, further comprising a drape across the interface on the proximal end of the main body, wherein the drape provides a sterile shield between the actuator and the surgical instrument and wherein preferably the sterile shield allows the user to couple or decouple the surgical instrument from the main body without compromising pre-established sterility.

15. A surgical robot comprising the robot arm coupled with the end effector of claim 1.
